# EUROPEAN PATENT APPLICATION

(11) **EP 4 810 601 A1**
(43) Date of publication of application: **23.09.2026**
(21) Application number: 24891847.6
(22) Date of filing: 13.11.2024
(51) Int. Cl.: C12N 5/0783, A61K 35/17, A61K 39/00, A61P 35/00

(54) **NOVEL POPULATION OF MARROW-INFILTRATING LYMPHOCYTES AND METHOD FOR PRODUCING THE SAME**

(30) Priority: 13.11.2023 KR 20230156399
(71) Applicant: Vaxcell-Bio Co., Ltd., Jeollanam-do 58141 (KR)
(72) Inventor: LEE, Je Jung, Gwangju 61513 (KR); VO, Manh Cuong, Hwasun-gun Jeollanam-do 58125 (KR); JUNG, Sung Hoon, Seoul 06509 (KR)
(74) Representative: Klöckner, Christoph
(86) International application number: PCT/KR2024/096464
(87) International publication number: WO 2025/105901

(57) **Abstract**

The present invention relates to a novel population of marrow-infiltrating lymphocytes (MILs) and a method for producing same. Specifically, the present invention provides a MIL cultivation method that enables the production of a novel MIL population with strong and long-lasting cytotoxicity against target cells. The novel population of MILs produced by the method can be used as a new cancer immunotherapy due to the high anticancer effect thereof.

## Description

### [Technical Field]

The present invention relates to a novel marrow-infiltrating lymphocytes (MILs) population and a method for producing the same. Specifically, the present invention provides a MIL culturing method capable of providing a novel MILs population having characteristics of potent and long-lasting cytotoxicity against target cells, and the novel MILs population prepared by the method can be used as a new cancer immunotherapy due to high anti-cancer effects.

### [Background Art]

Multiple myeloma(MM) is a disease that is still difficult to treat effectively, despite the development of various effective treatment methods including proteasome inhibitors, immunomodulators, or monoclonal antibodies. Patients who are completely cured of multiple myeloma are very rare, and most of patients with multiple myeloma are refractory to treatment or have relapsed multiple myeloma. Therefore, it is necessary to develop a new treatment for multiple myeloma that can overcome the problems.

Adoptive T-cell therapy, also known as cellular immunotherapy, has made significant progress in the treatment of multiple myeloma over the past decade through several approved chimeric antigen receptor(CAR)-T cell therapies. However, current adoptive immunotherapy uses peripheral blood lymphocytes(PBLs) as a T-cell source, and PBL lacks inherent tumor specificity, making them vulnerable to tumor escape and antigen loss, which is one of the major drawbacks of CAR-T cells produced by PBL. One of the methods to overcome these drawbacks and increase the tumor specificity of adoptive T-cell therapy is to use tumor-infiltrating lymphocytes (TILs) containing polyclonal memory T cells targeting various tumor-associated antigens obtained from solid tumors of cancer patients. However, TIL cannot be applied to all solid tumor patients. For example, so-called cold tumor patients with low immunogenicity lack TIL, and TIL therapy for solid tumors has impractical aspects due to various reasons, such as high costs because it requires a long expansion time and a high concentration of IL-2.

Regarding solid tumors, marrow-infiltrating lymphocytes(MILs), which are drawing attention as a new T-cell source for adoptive T-cell therapy, have been reported to have specificity for target cancer cells. Unlike TIL, MIL can be easily obtained from all multiple myeloma patients using a simple procedure and can be expanded rapidly, so MIL can be used as a source for producing multiple myeloma-specific T cells in adoptive T-cell therapy.

The inventors of the present invention have conducted various studies on adoptive T-cell therapy, especially on a T-cell source that can increase tumor specificity and be produced rapidly, and as a result, the present invention is completed by generating ex vivo expanded and activated MIL(eMIL) from multiple myeloma patients and proving its excellence by evaluating the immunological characteristics and cytotoxicity of such eMIL.

### [Prior Art Documents]

### [Non-Patent Documents]

(Non-Patent Documents 0001) Cowan AJ, Green DJ, Kwok M, Lee S, Coffey DG, Holmberg LA, et al. Diagnosis and Management of Multiple Myeloma: A Review. Jama. 2022;327(5):464-77.
(Non-Patent Documents 0002) Kyle RA RS. Treatment of Multiple Myeloma: A Comprehensive Review. Clin Lymphoma Myeloma. 2009;9(4):278-88. doi: 10.3816/CLM.2009.n.056.
(Non-Patent Documents 0003) Bonini C, Mondino A. Adoptive T-cell therapy for cancer: The era of engineered T cells. Eur J Immunol. 2015;45(9):2457-69.

### [Summary of Invention]

### [Technical Problem]

The present invention aims to provide a method for preparing a marrow-infiltrating lymphocytes population that can be rapidly expanded ex vivo and has activity against tumors.

The present invention aims to provide a marrow-infiltrating lymphocytes population having activity against tumors.

The present invention aims to provide a composition comprising a marrow-infiltrating lymphocytes population.

The present invention aims to provide a method for treating a multiple myeloma subject, comprising the step of administering a marrow-infiltrating lymphocytes population.

### [Technical Solution]

The present invention provides a method for culturing a marrow-infiltrating lymphocytes(MIL) population, comprising the steps of: (a) isolating MIL from the bone marrow of a multiple myeloma patient; and (b) culturing the isolated MIL by contacting the same with anti-CD3 and anti-CD28 antibodies in the presence of IL-2, IL-7, and IL-15.

The method according to the present invention is characterized in that the concentration of IL-2 used in step (b) is less than 200 IU/mL.

The period of culturing MIL during step (b) of the method according to the present invention may be 14 to 21 days.

The period of culturing MIL during step (b) of the method according to the present invention may be 14 days.

In the method according to the present invention, the cultured MIL population obtained in step (b) may have an increased ratio of CD8⁺ central memory T cells compared to the isolated MIL obtained in step (a).

In the method according to the present invention, the cultured MIL population obtained in step (b) may have an increased ratio of CD8⁺ central memory T cells and a decreased ratio of CD4⁺ T cells compared to the isolated MIL obtained in step (a).

In the method according to the present invention, the cultured MIL population obtained in step (b) may have a decreased ratio of regulatory T cells and myeloid-derived suppressor cells compared to the isolated MIL obtained in step (a).

In the method according to the present invention, the cultured MIL population obtained in step (b) may have decreased expression levels of TIM3 and CD73 compared to the isolated MIL obtained in step (a).

In the method according to the present invention, a T memory stem cell population of the isolated marrow-infiltrating lymphocytes population obtained in step (a) may be substantially and completely differentiated into memory T cells after 5 days of culture or thereafter.

The present invention provides an isolated MIL population, which is characterized by representing an increased ratio of CD8⁺ T cells and central memory T cells, a decreased ratio of CD4⁺ T cells, regulatory T cells, and myeloid-derived suppressor cells, and low expression levels of TIM3 and CD73.

The MIL population according to the present invention may represent a higher expression ratio of CD107a, that is, high cytotoxicity, against CD138⁺ primary multiple myeloma cells compared to an isolated peripheral blood lymphocyte population.

The present invention provides a composition comprising the MIL population prepared according to the present invention.

The composition according to the present invention may be a composition for use in the treatment of a subject with multiple myeloma.

The present invention provides a method for treating a subject with multiple myeloma, comprising the step of administering the MIL population prepared according to the present invention.

### [Advantageous Effects of Invention]

One of the important requirements of adoptive T-cell therapy is to obtain a sufficient amount of myeloma-specific T cells. Generally, peripheral blood lymphocyte has been used as a T-cell source for adoptive T-cell therapy because a large amount of lymphocytes can be easily obtained. However, peripheral blood lymphocyte obtained in this manner may have low tumor specificity. In multiple myeloma, the bone marrow is the site of disease onset and progression, as well as a secondary lymphoid organ rich in T-cell markers and antigen-presenting cells. The abundance of antigen-presenting cells in the bone marrow can allow them to be continuously exposed to malignant plasma cells, ultimately enabling the enrichment and maintenance of myeloma T cells with polyclonal antigen specificity compared to peripheral blood lymphocyte. According to the present invention, marrow-infiltrating lymphocyte obtained from the bone marrow can be successfully expanded and activated over a relatively short period.

Furthermore, the marrow-infiltrating lymphocytes cell population expanded by the method according to the present invention shows increased cytotoxicity compared to expanded peripheral blood lymphocyte. This was particularly prominent in experiments against CD138⁺ primary cells of autologous patients.

The persistence of the delivered T cells also plays an important role in achieving long-term effects in multiple myeloma patients receiving adoptive T-cell therapy. Recently, chimeric antigen receptor T cells have emerged as a powerful immunotherapeutic agent for multiple myeloma patients, but the duration of response is relatively short at 8.8 months. The marrow-infiltrating lymphocytes cell population expanded by the method according to the present invention is characterized by a high ratio of CD8⁺ Tcm, and such an increase in the CD8⁺ Tcm ratio shows that marrow-infiltrating lymphocyte may have long-lasting cytotoxicity in the body.

The marrow-infiltrating lymphocytes cell population provided according to the present invention persists in the long term because it is tumor antigen-specific and rich in Tcm. Due to these characteristics, the marrow-infiltrating lymphocytes cell population provided according to the present invention is useful for immunotherapy and can be a promising platform for chimeric antigen receptor T-cell therapy instead of peripheral blood lymphocyte.

### [Brief Description of Drawings]

FIG. 1 is a schematic diagram showing a method for preparing ex vivo expanded and activated MIL.
FIG. 2 is a graph showing the increase of MIL over time.
FIG. 3 is a graph showing the expansion fold of MIL.
FIG. 4 is a graph showing the results of flow cytometry analysis of CD8⁺ and CD4⁺ T cells over time.
FIG. 5 is a graph showing changes in CD8⁺ and CD4⁺ T cells over time.
FIG. 6 is a graph showing the increase of central memory T cells over time after ex vivo cultivation of MILs.
FIG. 7 is a graph showing changes in central memory T cells over time after ex vivo cultivation of MILs.
FIG. 8 is a graph showing differentiation from T memory stem cells (CD62L+ CD95+ cells) within naive T cells (CD62L+ CD45RA+ cells) to central memory T cells (CD62L+ CD45RA- cells) after ex vivo cultivation of MILs.
FIG. 9 is a graph showing changes in the ratio of T memory stem cells (CD62L+ CD95+ cells) and central memory T cells (CD62L+ CD45RA- cells) over time after ex vivo cultivation of MILs.
FIG. 10 is a graph showing changes in the expression ratio of immune checkpoint molecules on the surface of MILs proliferated after ex vivo cultivation of MILs.
FIG. 11 is a graph showing a decrease in the expression ratio of immune checkpoint molecules on the surface of MILs proliferated after ex vivo cultivation of MILs.
FIG. 12 is a graph showing changes in the expression ratio of immune checkpoint molecules expressed on the surface of target cancer cells after co-cultivation of cancer cells and ex vivo proliferated MILs.
FIG. 13 is a graph showing changes in the expression ratio of immune checkpoint molecules expressed on the surface of target cancer cells after co-cultivation of cancer cells and ex vivo proliferated MILs.
FIG. 14 is a graph showing a decrease in the ratio of regulatory T cells (Tregs) within proliferated MILs over time after ex vivo cultivation of MILs.
FIG. 15 is a graph showing a decrease in the ratio of myeloid-derived suppressor cells (MDSC) within proliferated MILs over time after ex vivo cultivation of MILs.
FIG. 16 is a graph showing changes in IFN-γ production of ex vivo proliferated MILs by stimulation of target cancer cells and an increase in IFN-γ production of ex vivo proliferated MILs by stimulation of autologous cancer cells isolated from patients.
FIG. 17 is a graph showing CD107a expression analysis, which indicates a significant increase in cytotoxicity of ex vivo proliferated MILs against autologous cancer cells isolated from patients.
FIG. 18 is a graph showing CD107a expression change, which indicates a significant increase in cytotoxicity of ex vivo proliferated MILs against autologous cancer cells isolated from patients.
FIG. 19 is a graph showing a significant increase in cytotoxicity of ex vivo proliferated MILs for 14 days against target cancer cells.
FIG. 20 is a graph showing an increase in cytotoxicity of ex vivo proliferated MILs for 21 days against target cancer cells.
FIG. 21 is a graph showing the ratio of surviving cancer cells after 20 hours of co-cultivation to confirm the cytotoxicity of ex vivo proliferated MILs for 14 days against the U226 cancer cell line.
FIG. 22 is a graph showing the ratio of surviving cancer cells after 20 hours of co-cultivation to confirm the cytotoxicity of ex vivo proliferated MILs for 21 days against the U226 cancer cell line.
FIG. 23 is a graph showing the ratio of surviving cancer cells after 20 hours of co-cultivation to confirm the cytotoxicity of ex vivo proliferated MILs for 21 days against the RPMI8226 cancer cell line.

### [Best Mode for Carrying Out the Invention]

Hereinafter, the present invention and examples will be described in detail with reference to the accompanying drawings so that those of ordinary skill in the art to which the present invention pertains can easily implement the same. However, the present invention may be embodied in many different forms and is not limited to the embodiments and examples described herein.

The immune cells of the present invention may refer to cells of hematopoietic origin that are functionally involved in the initiation and/or execution of innate and/or adaptive immune responses. The immune cells of the present invention may be derived from stem cells. The stem cells may be adult stem cells, non-human embryonic stem cells, non-human stem cells, umbilical cord blood stem cells, progenitor cells, bone marrow stem cells, induced pluripotent stem cells, pluripotent stem cells, or hematopoietic stem cells.

The immune cells may be, but are not limited to, leukocytes, neutrophils, eosinophils, basophils, monocytes, lymphocytes, T cells, cytotoxic T cells, natural killer T cells, dendritic cells, or a combination thereof.

As used herein, the term "marrow infiltrating lymphocyte (MIL)" refers to lymphocytes derived from bone marrow. In the present specification, the term "MIL" may mean eMIL depending on the context. Marrow infiltrating lymphocytes have distinguishable differences from tumor infiltrating lymphocytes("TIL") as well as peripheral blood lymphocytes. The bone marrow microenvironment corresponds to a special immunological niche due to the abundance of antigen-presenting cells. The presence of these antigen-presenting cells leads to the maintenance of higher levels of central memory cells, such as those where antigen processing and presentation are found in the bone marrow compartment (Li JM et al J Immunol. 2009 Dec 15;183(12):7799-809). These MIL express markers of memory T cells, such as CD45RO⁺ and CD62L⁺, and have a greater population of memory cells than those found in the peripheral blood lymphocyte population (Noonan K et al Clin Cancer Res. 2012 Mar 1;18(5):1426-34). Furthermore, MIL is not merely the "TIL" of hematological malignancies because of its ability to continuously perform priming of memory cells to antigens (Beckhove P et al J Clin Invest. 2004 Jul 1; 114(1):67-76, Castiglioni P et al J Immunol 2008; 180:4956-4964). Unlike TIL, MIL can be harvested and expanded in all patients (Noonan, K et al Sci. Transl Med. 2015 May 20; 7(288): 288ra78). TIL is found in only about 50% of patients, and only about 25% of patients contain expandable TIL. In contrast to peripheral blood lymphocyte, MIL possesses a broad endogenous antigen repertoire, accounting for its inherent tumor specificity not present in PBL (Noonan et al Clin Cancer Res.).

The source of MIL for use in the cultivation method of the MIL population according to the present invention can be obtained from a patient having any of a number of types of cancer, including hematological malignancy and solid tumor, preferably from a multiple myeloma patient. Preferably, it can be obtained from bone marrow having increased tumor specificity compared to peripheral blood.

For the isolated MIL cell, activated MIL can be generated by activating and expanding bone marrow cell in vitro by performing a method such as contacting with anti-CD3 and anti-CD28 antibodies, and the contacting can use, for example, anti-CD3 and anti-CD28 antibody-coated magnetic beads. The process of contacting the MIL cell with anti-CD3 and anti-CD20 antibodies is performed in the presence of IL-2, IL-7, and/or IL-15, preferably under an environment where IL-2, IL-7, and IL-15 are all present.

In the present invention, the step of cultivating the isolated MIL cell is preferably performed in the presence of a low concentration of IL-2, and the low concentration is preferably less than 200 IU/mL, and more preferably less than 100 IU/mL. If the concentration of IL-2 is 200 IU/mL or more, the ratio of central memory CD8⁺ T cell in the MIL cell population may decrease. The step of cultivating the isolated MIL cell in the present invention may be performed, for example, in the presence of 100 IU/mL of IL-2, 10 ng/mL of IL-7, and 10 U/mL of IL-15.

In the present invention, the step of cultivating the isolated MIL cell can be performed for 14 days to 21 days, and is preferably performed for 14 days. The inventors of the present invention confirmed that the ratio of central memory CD8⁺ T cell reached 80% on the 14th day of cultivating the MIL cell isolated according to the method according to the present invention. As such, a MIL cell population having such a high ratio of central memory CD8⁺ T cell has never been suggested conventionally.

The MIL cultivation method according to the present invention can provide a MIL cell population in which the ratio of CD8⁺ central memory T cell is increased and the ratio of CD4⁺ T cell is decreased, compared to a MIL population just isolated from a patient. For example, the MIL population cultivated by the method according to the present invention may have a ratio of CD8⁺ central memory T cell of 30% or more, 40% or more, 50% or more, 60% or more, 70% or more, or 80% or more, and a ratio of CD4⁺ T cell of 60% or less, 50% or less, 40% or less, 30% or less, 20% or less, or 10% or less.

The MIL cultivation method according to the present invention can provide a MIL cell population in which the ratio of regulatory T cell and myeloid-derived suppressor cell is decreased, compared to a MIL population just isolated from a patient. For example, the MIL population cultivated by the method according to the present invention may have a ratio of regulatory T cell of 15% or less, 10% or less, 5% or less, or 1% or less, and a ratio of myeloid-derived suppressor cell of 15% or less, 10% or less, 5% or less, or 1% or less.

The MIL cultivation method according to the present invention can provide a MIL cell population in which expression levels of TIM3 and CD73 are decreased, compared to a MIL population just isolated from a patient. For example, the MIL cell population cultivated by the method according to the present invention may have an expression rate of TIM3 of 30% or less, 20% or less, or 10% or less, and an expression rate of CD73 of 40% or less, 30% or less, 20% or less, or 10% or less.

The MIL cultivation method according to the present invention can provide a MIL cell population in which the T memory stem cell population of the MIL population just isolated from a patient is substantially and completely differentiated into central memory T cell after 5 days of cultivation or thereafter.

The isolated marrow infiltrating lymphocyte population according to the present invention is characterized by exhibiting an increased ratio of CD8⁺ T cell and central memory T cell, a decreased ratio of CD4⁺ T cell, regulatory T cell, and myeloid-derived suppressor cell, and a low expression level of TIM3 and CD73; for example, it is characterized by exhibiting an increased ratio of CD8⁺ T cell and central memory T cell, a decreased ratio of CD4⁺ T cell, regulatory T cell, and myeloid-derived suppressor cell, and a low expression level of TIM3 and CD73 compared to a MIL population just isolated from a patient.

As used herein, the term "differentiation" refers to a developmental process by which a cell becomes specialized for a particular function, for example, acquiring morphological characteristics and/or functions.

As used herein, the term "substantially and completely differentiated" means that a cell is finally differentiated into a mature and fully differentiated cell, and means that 90% or more, 95% or more, or 100% of the cell is differentiated.

As used herein, the term "memory T cell" refers to a type of T cell, which means an antigen-specific T cell that remains for a long period even after an antigen is removed, and refers to a cell that responds to the antigen by rapidly converting into an effector T cell upon re-exposure to the specific antigen.

As used herein, the term "regulatory T cell" refers to a type of T cell, which performs a function of maintaining homeostasis and self-tolerance by suppressing an immune response, and is also referred to as T_{reg}.

As used herein, the term "T memory stem cell" refers to a type of memory T cell, which has characteristics such as a long lifespan, continuous self-renewal, rapid differentiation into an effector T cell, and resistance to apoptosis, and is also referred to as T_{scm}.

As used herein, the term "myeloid-derived suppressor cell" refers to a type of immature myeloid cell that suppresses both innate and adaptive immune response, and is also referred to as MDSC.

As used herein, the term "central memory T cell" has a high self-renewal capacity and primarily exists in a lymph node and a peripheral circulation system.

As used herein, the term "CD4⁺" means that CD4(cluster of differentiation 4), which is a glycoprotein serving as a co-receptor for a T-cell receptor(TCR), exists on a surface of a cell.

As used herein, the term "CD8⁺" means that CD8(cluster of differentiation 8), which is a glycoprotein serving as a co-receptor for a T-cell receptor (TCR), exists on a surface of a cell.

As used herein, the term "cancer" may specifically be one or more selected from the group consisting of brain tumor, cervical cancer, ovarian cancer, prostate cancer, lung cancer, cholangiocarcinoma, kidney cancer, stomach cancer, liver cancer, retinoblastoma, choriocarcinoma, small intestine cancer, colon and rectal cancer, non-small cell lung cancer, gastric adenocarcinoma, acute lymphocytic leukemia, acute myeloid leukemia, breast cancer, bone sarcoma, bladder cancer, anaplastic astrocytoma, and multiple myeloma, but is not limited thereto.

As used herein, the term "multiple myeloma(MM)" refers to a type of hematological malignancy caused by abnormal differentiation and proliferation of a plasma cell of bone marrow.

As used herein, the term "prevention" refers to any act of suppressing or delaying the onset of a disease by administration of a composition, and "treatment" refers to any act of improving or advantageously changing a symptom of a subject suspected of or having a disease by administration of a composition.

A dosage of the MIL cell population according to the present invention can be appropriately selected by those skilled in the art depending on a condition, body weight, disease, formulation, administration route, and period of a subject.

The MIL cell population according to the present invention can be administered through any general route to reach a target tissue. For example, it can be administered intravenously, subcutaneously, or intraperitoneally, but is not limited thereto.

As used herein, the term "co-administration" refers to administering two or more types of active ingredients simultaneously or sequentially.

The MIL cell population according to the present invention can be co-administered with another anticancer agent appropriately selected by those skilled in the art. For example, it can be co-administered with an immune checkpoint inhibitor, but is not limited thereto.

It can be co-administered with an immune checkpoint inhibitor, for example, tiragolumab, that inhibits TIGIT, which is an immune checkpoint protein confirmed to increase in amount on a surface of a tumor cell upon contact with the MIL cell population cultivated according to the present invention, but is not limited thereto.

It can be co-administered with an immune checkpoint inhibitor, for example, oleclumab, that inhibits CD73, which is an immune checkpoint protein confirmed to increase in amount on a surface of a tumor cell upon contact with the MIL cell population cultivated according to the present invention, but is not limited thereto.

Hereinafter, the present invention will be described in more detail through an example, but the following example is for illustrative purposes only and is not intended to limit the scope of the present invention.

### [Preparation Example 1]

### Production of ex vivo expanded and activated MILs(eMIL)

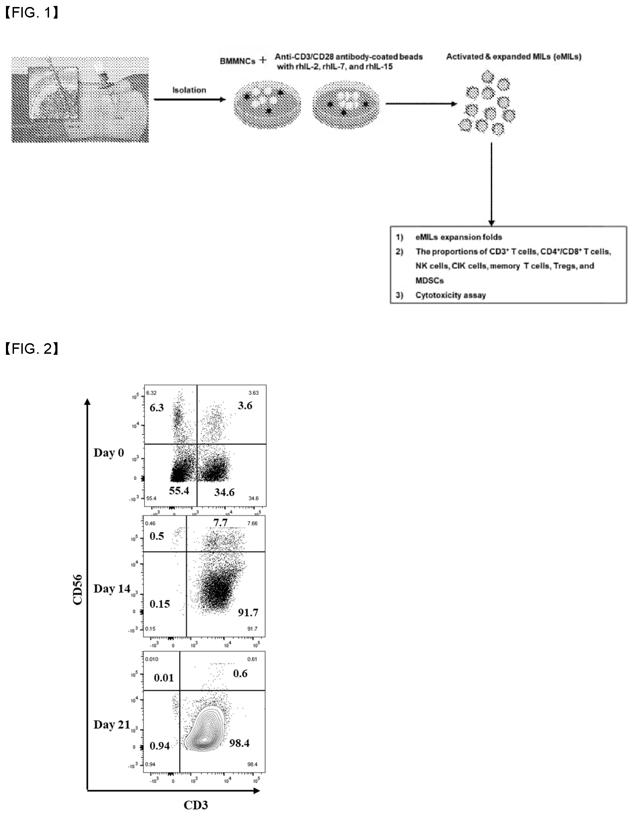

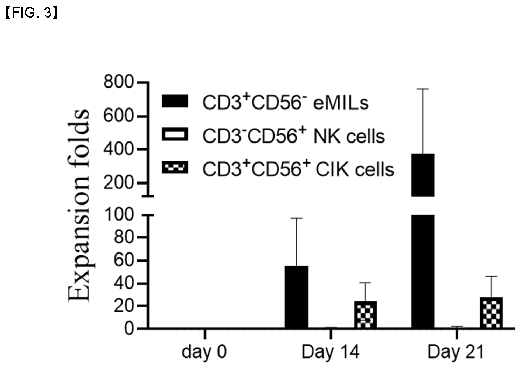

The ex vivo expanded and activated MIL(eMIL) was producted by the same method as in FIG. 1. Specifically, bone marrow mononuclear cells (BMMNCs) were isolated from bone marrow of a multiple myeloma patient using density gradient centrifugation with Ficoll-Hypaque (d = 1.077, Lymphoprep^{™}; Axis-Shield, Oslo, Norway). Thereafter, the BMMNCs were co-cultivated with anti-CD3/CD28 antibody-coated beads in a 24-well plate in RPMI 1640 medium containing 10% FBS, 100 U/mL penicillin, 100 µg/mL streptomycin, 4 mmol/L L-glutamine, 100 U/mL recombinant human IL-2, 10 ng/mL IL-7, and 10 U/mL IL-15. The medium was replaced every 2 to 3 days, and the eMIL was continuously cultivated for up to 21 days. As shown in FIGS. 2 and 3, the eMIL continuously increased for 21 days, and the median expansion rate on day 14 of cultivation compared to day 0 of cultivation was 55.7%, and the median expansion rate on day 21 was 374.3, showing a significant increase.

### [Example 1]

### Immune population analysis of ex vivo expanded and activated MILs(eMIL)

To analyze the immune cell population of the eMIL, the eMIL prepared by the method of Preparation Example 1 was treated with a fluorescence-labeled monoclonal antibody, a sample was collected using a flow cytometer, and then expression of a cell surface marker was analyzed to confirm the composition of the immune cell population.

Specifically, to analyze CD3⁺ cells, NK cells(Natural killer cell), CIK cells(cytokine-induced killer cells), MDSC(myeloid-derived suppressor cells), regulatory T cells(T_{reg}), CD4⁺ T cells, CD8⁺ T cells, naïve T cells, central memory T cells(T_{cm}), TEMRA cells(terminally differentiated effector T cells), and Tₑₘ cells(effector memory T cells), eMIL(2 x 10⁵ cells) were washed with FACS buffer and treated with an Fc blocker. Thereafter, the cells were stained with surface monoclonal antibodies such as CD3-FITC, CD56-PE-Cy7, CD4-PE, CD8-Amyan, CD62L-PE-Cy7, CD45RA, CD33-Pacific blue, CD11b-Amyan, and PD-1-Pacific at 4°C for 30 minutes, and then washed and permeabilized using FACSTM Permeabilizing Solution 2 (BD Bioscience) at room temperature for 30 minutes. After collecting the prepared samples using a BD FACS Canto II(Becton Dickinson, Mountain View, CA, USA), the expression of cell surface markers was analyzed using Flow Jo software(TreeStar, San Carlos, CA, USA) to identify the composition of the immune cell populations.

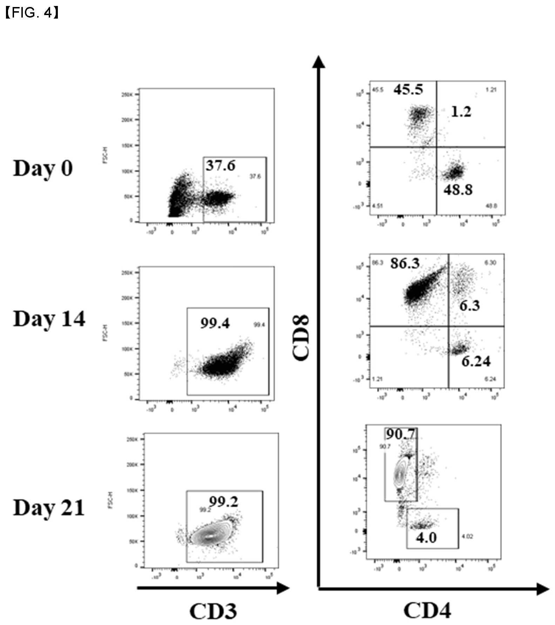

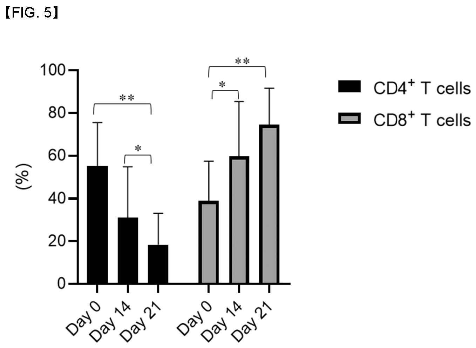

As a result, as shown in FIGS. 4 to 5, it was confirmed that on the first day of culture (Day 0), the most of CD3⁺ MILs consisted of CD4⁺ T cells (55.2%), and CD8⁺ T cells accounted for 39%. On Day 14 and Day 21 of culture, the proportions of CD4⁺ T cells were 31% and 18.4%, respectively, while the proportions of CD8⁺ T cells were 59.8% on Day 14 and 74.6% on Day 21.

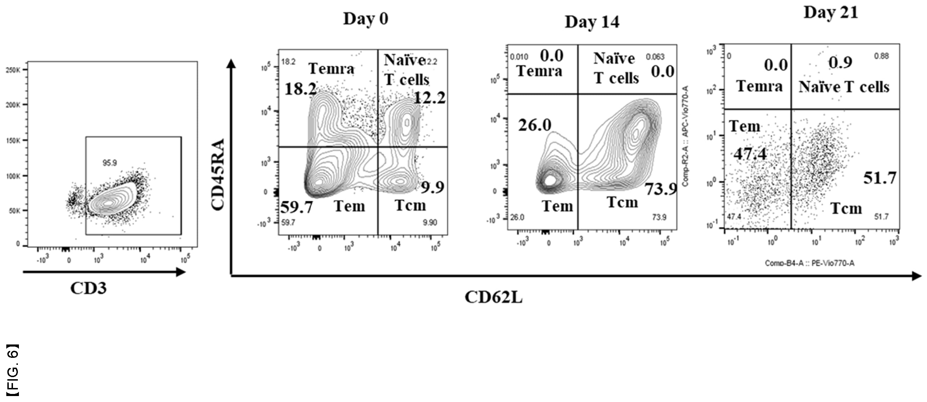

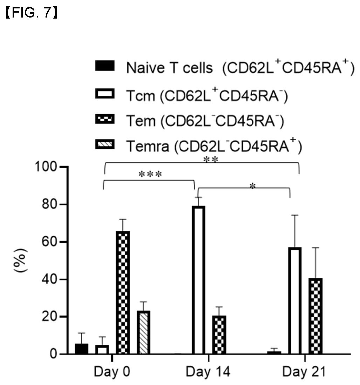

In addition, as shown in FIGs. 6 to 7, it was confirmed that on the first day of culture (Day 0), BMMNC included 60% of Tₑₘ (CD62L⁻CD45RA⁻), 12% of naïve T cells (CD62L⁺CD45RA⁺), 10% of T_{cm} (CD62L⁺CD45RA⁻), and 18% of TEMRA (CD62L⁻CD45RA⁺). Thereafter, it was confirmed that on day 14 of culture, the proportion of T_{cm} was increased to 80% while the proportion of Tₑₘ was 20%, and on day 21 of culture, the proportion of T_{cm} was 60% on average and the proportion of Tₑₘ was 40% on average. Furthermore, it was confirmed that on day 14 and day 21, the populations of naïve T cell and TEMRA cell were not detected.

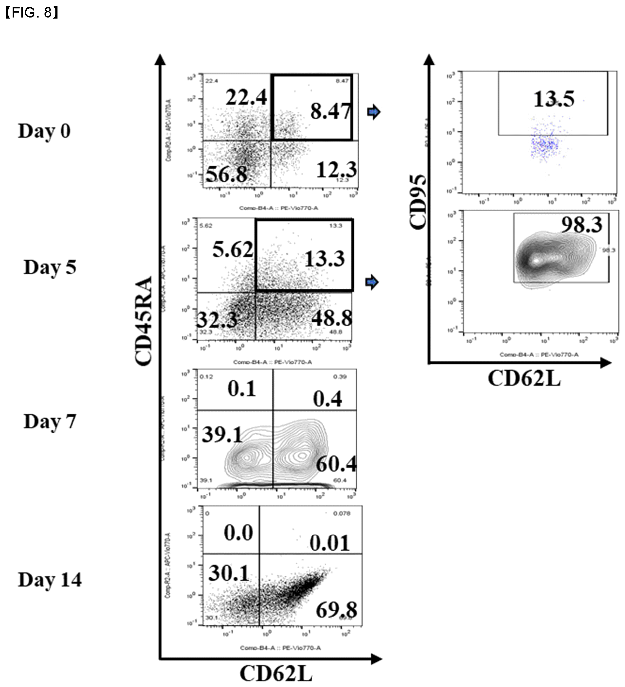

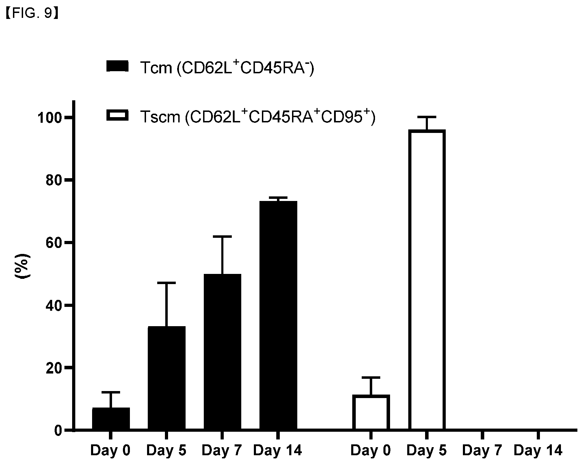

As shown in FIGs. 8 to 9, in the BMMNC, the proportion of T memory stem cell (T_{scm}; CD62L⁺CD45RA⁺CD95⁺) was 13.5% on the first day (Day 0) of culture, and when the new MIL culture technology was used, the proportion of T_{scm} was increased to nearly 100% on day 5, and the T_{scm} cell population was completely differentiated into T_{cm} from day 7 of culture. In addition, compared to day 14 of culture, it was confirmed that T_{cm} was decreased on day 21, and it was confirmed that the optimal time for obtaining the eMIL was day 14 of culture.

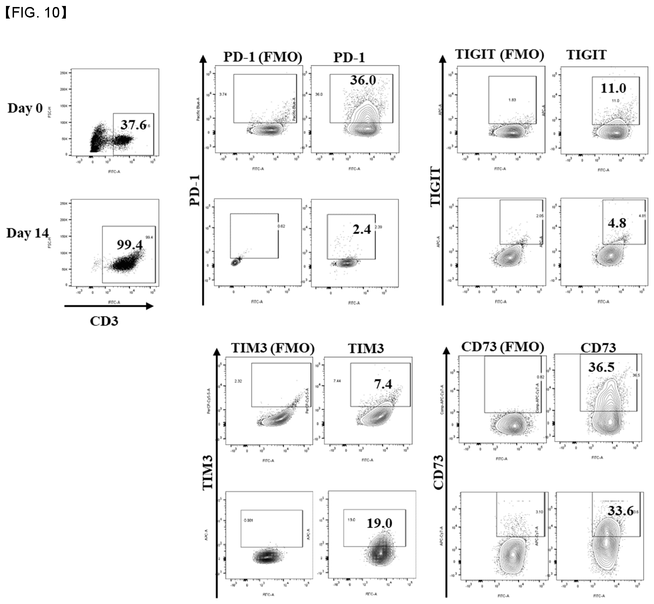

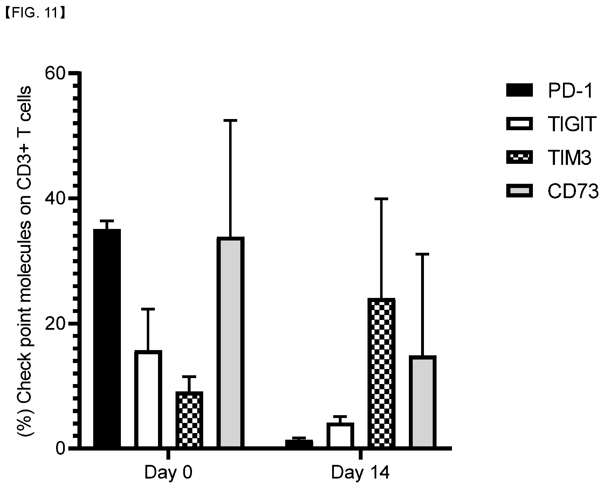

Additionally, as shown in FIGS. 10 and 11, on day 14 of culture, the CD3⁺ T cell showed low expression levels of PD-1 and TIGIT (1.2% and 4.8%, respectively), but showed high expression levels of TIM3 and CD73 (21% and 33%, respectively).

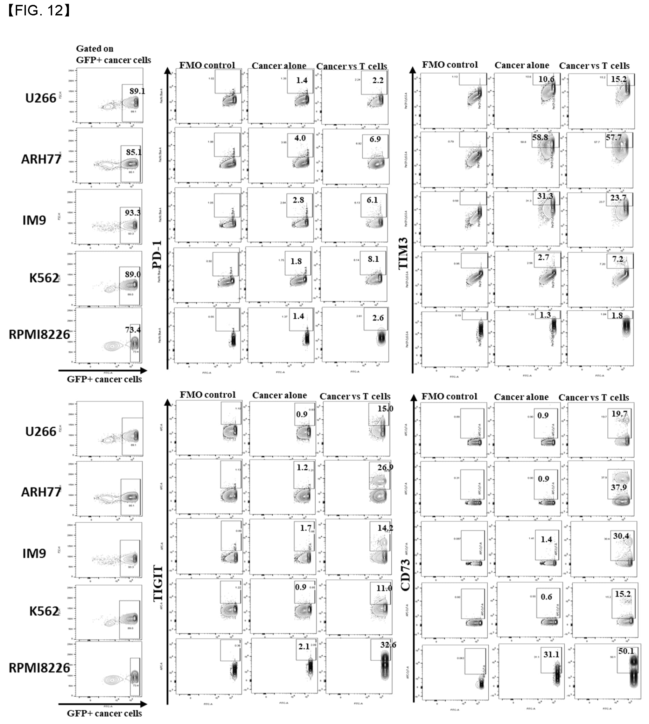

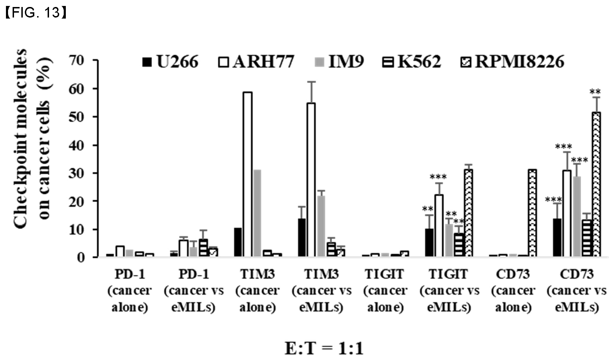

Furthermore, as shown in FIGS. 12 to 13, when cancer cell lines(U266, ARH77, IM9, RPMI8226, and K562) were co-cultured with the eMIL, it was confirmed that the expression of TIGIT and CD73 in the cancer cells was significantly increased. In the absence of the eMIL, all cancer cells showed negative expression of TIGIT, and most of the cancer cells(U266, ARH77, IM9, and K562) showed negative expression of CD73.

It was confirmed that although the expression of TIGIT and CD73 checkpoint molecules was increased in the eMIL and cancer cells after co-culture, the eMIL having a high proportion of CD8⁺ T_{cm} cell was successfully generated from the BMMNC of a multiple myeloma patient.

### [Example 2]

### Analysis of cell proportions of MDSC and Treg in eMIL population

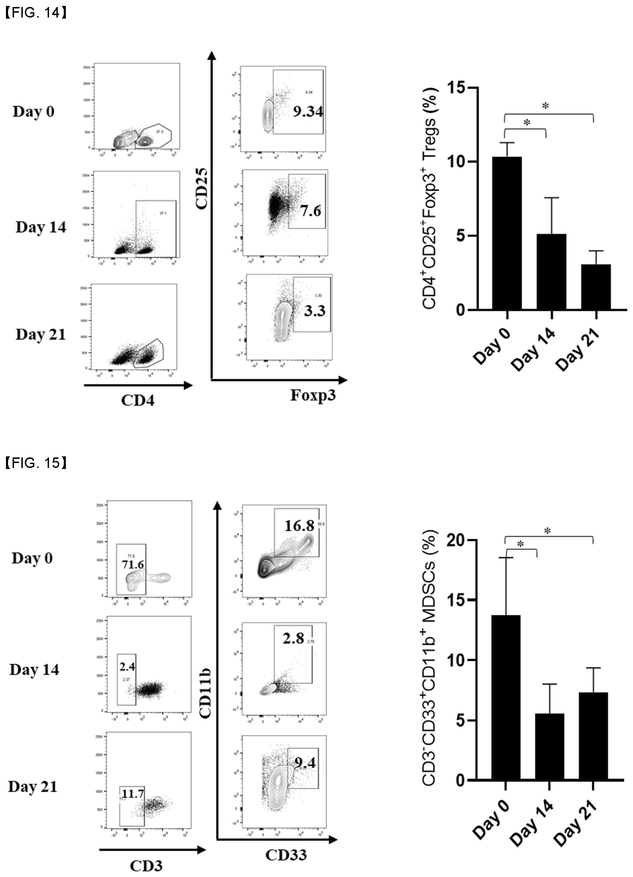

An immune population analysis of the eMIL was performed in the same manner as in Example 1. As a result, as shown in FIG. 14, an expression proportion of a CD3⁺CD4⁺CD25⁺Foxp3⁺ regulatory T cell (T_{reg}) was dramatically decreased on day 14 and day 21 of culture, and as shown in FIG. 15, a proportion of a CD3⁻CD33⁺CD11b⁺ MDSC(myeloid-derived suppressor cells) was also decreased.

Therefore, it was confirmed that the immune population of the eMIL prepared by the method of the present invention contains almost no immunosuppressive cell (T_{reg} and MDSC).

### [Example 3]

### Cytotoxicity analysis of eMIL

To confirm whether the eMIL effectively kills multiple myeloma cells, functional differences between the eMIL and an activated peripheral blood lymphocyte(ePBL) were experimented. Specifically, to evaluate a killing capacity of the eMIL, an IFN-γ ELISA, an LDH assay, an IncuCyte analysis, and a CD107a degranulation assay were performed.

### 3-1. Tumor-specific cytotoxicity of eMIL: IFN-γ ELISA analysis

The IFN-γ ELISA analysis was performed as follows. 5×10⁴ cells of the eMIL and the ePBL were cultured without or with 5×10⁴ target cells (K562, U266, RPMI8226, ARH77, and IM9) in a 96-well U-bottom plate. After 24 hours, a supernatant was collected, and a production amount of IFN-γ was measured using an ELISA kit(BD Biosciences) according to the manufacturer's protocol. The production amount was determined by analyzing each sample in triplicate and calculating a mean absorbance for each set of standards and sample analyses.

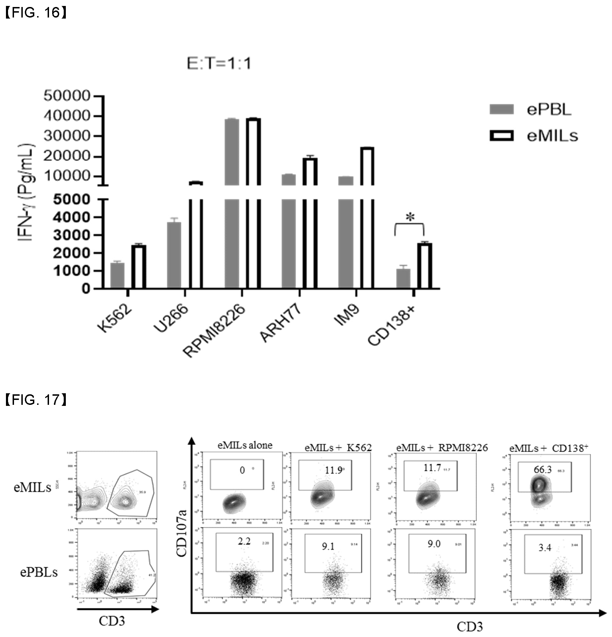

As shown in FIG. 16, on day 14 of culture, IFN-γ production level of the eMIL was slightly increased against cancer cell lines (K562, U266, ARH77, and IM9) compared to an ePBL control group, whereas an IFN-γ production level of the eMIL against CD138⁺ autologous primary tumor cells isolated from a multiple myeloma patient was significantly increased compared to the ePBL control group.

### 3-2. Tumor-specific cytotoxicity of eMIL: IncuCyte analysis and CD107a degranulation assay

The IncuCyte analysis was performed as follows. Multiple myeloma cell lines (U266 and RPMI8226) were transduced with a green fluorescent protein(GFP)-lentivirus, and then GFP-positive multiple myeloma cells were sorted by FACS. For a kinetics analysis of tumor cell death, each GFP-transduced cell line(U266 and RPMI8226) was pre-stained with 2 µg of an anti-human HLA-A, B, or C antibody(clone: W6/32, Biolegend). Thereafter, 10⁶ cells were added to 100 µL of an RPMI medium and cultured for 20 minutes, followed by centrifugation and washing. Each GFP-transduced cell line was individually seeded into a 96-well U-bottom plate at a concentration of 1×10⁴ cells per well, and on the next day, the eMIL was added at an effector:target(E:T) ratio in a range of 1:1. The number of viable target cells was observed through hourly fluorescence imaging over 72 hours using an IncuCyte Live Cell Analysis System(Sartorius). The number of living cells was quantified with IncuCyte S3 software (Sartorius) and normalized to the number of living cells in a control group containing only target cells without treatment of the eMIL.

The CD107a degranulation assay was performed as follows. 5×10⁴ cells of the eMIL and the ePBL were cultured without or with 5×10⁴ target cells(K562, U266, RPMI8226, ARH77, IM9, and CD138⁺ multiple myeloma cells isolated from a patient) in a 96-well U-bottom plate. The culture was performed with 5 µL of a PE-conjugated anti-human CD107a antibody in a 96-well U-bottom plate without or with 5×10⁴ target cells(K562, U266, RPMI8226, ARH77, IM9, and CD138⁺ primary multiple myeloma cells). After 1 hour, Monensin and brefeldin A (BD Biosciences) were added, followed by additional culture for 4 hours. Thereafter, after staining with an anti-human CD3 antibody, cells were harvested.

As shown in FIGS. 17 to 18, on day 14 of culture, the eMIL showed a higher CD107a⁺ positive population against the CD138⁺ multiple myeloma cells isolated directly from the patient, whereas it was not the case in the target cell lines. In contrast, the ePBL showed a lower CD107a⁺ positive population when co-cultured with the cancer cell lines and the primary CD138+ multiple myeloma cells isolated from the patient.

### 3-3. Tumor-specific cytotoxicity of eMIL: LDH assay

The LDH assay was performed as follows. For target cells(K562, U266, RPMI8226, ARH77, IM9, and CD138⁺ multiple myeloma cells), 10⁶ cells were added to 100 µL of an RPMI medium containing 2 µg of an anti-human HLA-A, B, or C antibody(clone: W6/32, Biolegend, USA), cultured for 20 minutes, and then washed through centrifugation. Thereafter, the target cells were co-cultured with the eMIL at a 1:1 ratio in a Costar 96-well plate(Corning, USA) at 37°C with 5% CO₂ for 6 hours. Finally, a supernatant was collected to measure a concentration of LDH, a cytoplasmic enzyme released upon cell lysis, and the concentration of LDH was measured using a CytoTox 96 non-radioactive cytotoxicity assay(Promega, USA), and a cell lysis rate was calculated according to the manufacturer's protocol.

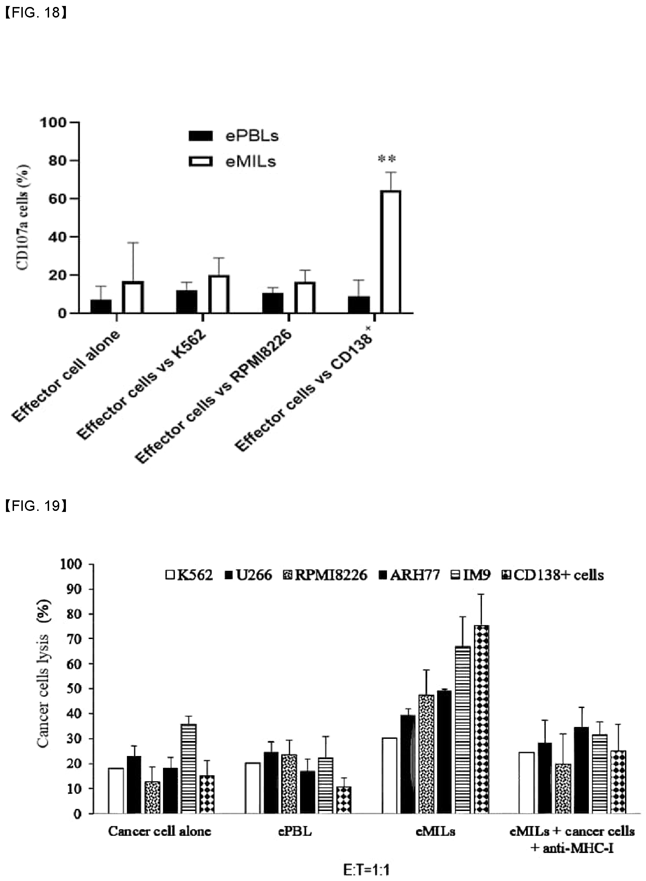

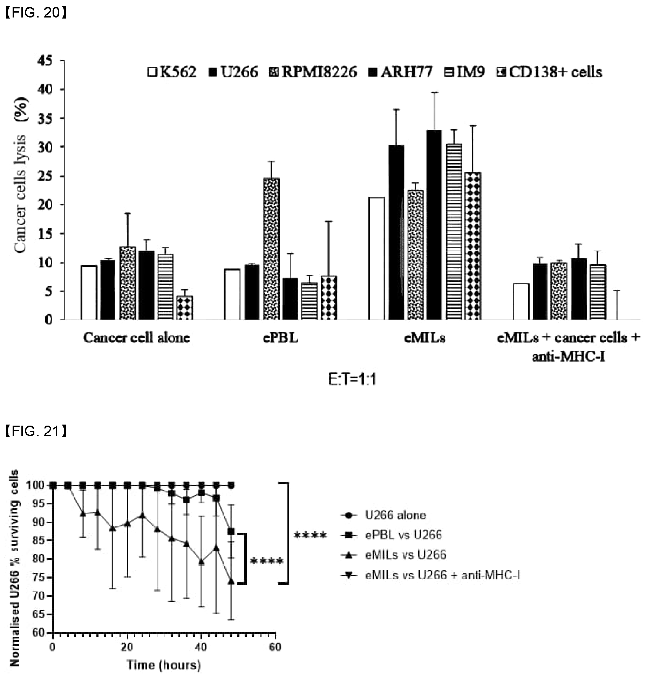

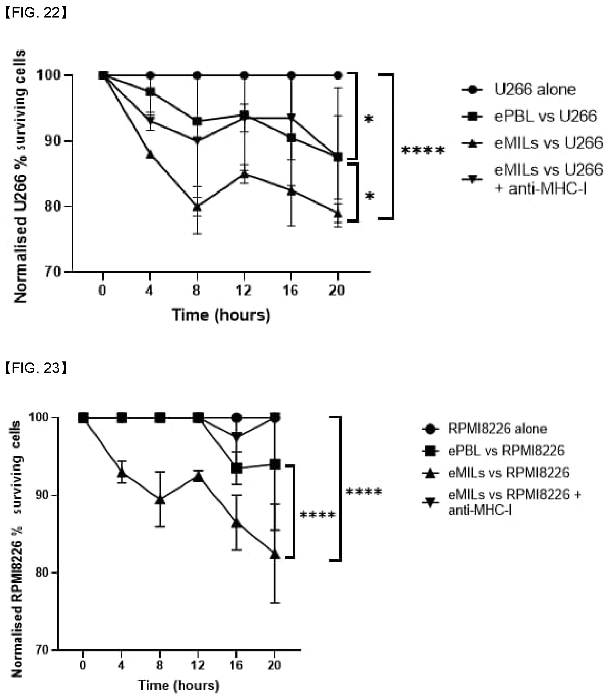

As a result of calculating the cell lysis rate after culturing the eMIL and the ePBL with target leukemia(K562) and target myeloma cells(ARH77, U266, RPMI8226, IM9, and CD138⁺ primary multiple myeloma cells), as shown in FIGS. 19 to 23, the eMIL exhibited greater cytotoxicity in all cases, particularly against the CD138⁺ primary multiple myeloma cells isolated from the patient. Notably, it was confirmed that blocking the MHC class I antibody completely abolished the cytotoxic response of the activated eMIL against the CD138⁺ primary multiple myeloma cells.

As shown in FIGS. 24 and 25, the cytotoxicity of the eMIL against multiple myeloma cells was higher on day 14 than on day 21 of culture, and according to the data, it was confirmed that the optimal time for harvesting the eMIL using the new MIL culture technology was day 14 of culture.

Finally, as shown in Examples 3-1 to 3-3, it was confirmed that the eMIL was successfully produced from the BMMNC of the multiple myeloma patient according to the manufacturing method of the present invention and was verified to be functional.

## Claims

1. A method for culturing an isolated marrow-infiltrating lymphocyte population, comprising the steps of:
(a) isolating marrow-infiltrating lymphocytes (MILs) from bone marrow of a multiple myeloma patient; and
(b) culturing the isolated marrow-infiltrating lymphocytes by contacting the same with an anti-CD3 and an anti-CD28 antibodies in the presence of IL-2, IL-7, and IL-15.

2. The method for culturing an isolated marrow-infiltrating lymphocyte population according to claim 1, wherein a concentration of the IL-2 is less than 200 IU/mL.

3. The method for culturing an isolated marrow-infiltrating lymphocyte population according to claim 1 or 2, wherein the marrow-infiltrating lymphocytes are cultured for 14 to 21 days in step (b).

4. The method for culturing an isolated marrow-infiltrating lymphocyte population according to claim 1 or 2, wherein the marrow infiltration lymphocytes are cultured for 14 days in step (b).

5. The method for culturing an isolated marrow-infiltrating lymphocyte population according to claim 1 or 2, wherein the cultured marrow-infiltrating lymphocyte population obtained in step (b) has an increased ratio of CD8⁺ central memory T cells compared to the isolated marrow infiltration lymphocyte population obtained in step (a).

6. The method for culturing an isolated marrow-infiltrating lymphocyte population according to claim 1 or 2, wherein the cultured marrow-infiltrating lymphocyte population obtained in step (b) has an increased ratio of CD8⁺ central memory T cells and a decreased ratio of CD4⁺ T cells compared to the isolated marrow infiltration lymphocyte population obtained in step (a).

7. The method for culturing an isolated marrow-infiltrating lymphocyte population according to claim 1 or 2, wherein the cultured marrow-infiltrating lymphocyte population obtained in step (b) has a decreased ratio of regulatory T cells and myeloid-derived suppressor cells compared to the isolated MIL population obtained in step (a).

8. The method for culturing an isolated marrow-infiltrating lymphocyte population according to claim 1 or 2, wherein the cultured marrow-infiltrating lymphocyte population obtained in step (b) has decreased expression levels of TIM3 and CD73 compared to the isolated marrow infiltration lymphocyte population obtained in step (a).

9. The method for culturing an isolated marrow-infiltrating lymphocyte population according to claim 1 or 2, wherein a T memory stem cell population of the isolated marrow infiltration lymphocyte population obtained in step (a) is substantially and completely differentiated into central memory T cells after 5 days of culture or thereafter according to step (b).

10. An isolated marrow-infiltrating lymphocyte population, representing an increased ratio of CD8⁺ T cells and central memory T cells, a decreased ratio of CD4⁺ T cells, regulatory T cells, and myeloid-derived suppressor cells, and low expression levels of TIM3 and CD73.

11. The isolated marrow-infiltrating lymphocyte population according to claim 10, representing a higher expression ratio of CD107a against CD138⁺ primary multiple myeloma cells compared to an isolated peripheral blood lymphocyte population.

12. A composition comprising the isolated marrow-infiltrating lymphocyte population according to claim 10 or 11.

13. The composition of claim 12, wherein the composition is for use in treatment of a subject with multiple myeloma.

14. A method for treating a subject with multiple myeloma, comprising the step of administering the isolated marrow-infiltrating lymphocyte population according to claim 10 or 11 to the subject.
